# EUROPÄISCHE PATENTANMELDUNG

(11) **EP 4 813 356 A1**
(43) Veröffentlichungstag der Anmeldung: **30.09.2026**
(21) Anmeldenummer: 26166205.0
(22) Anmeldetag: 19.03.2026
(51) Int. Cl.: A61F 7/00

(54) **HYPOTHERMIEGERÄT UND VERFAHREN ZUR BEFÜLLUNG UND ENTLEERUNG EINES HYDRAULIKKREISLAUFS**

(30) Priorität: 27.03.2025 DE 102025111996
(71) Anmelder: Lauda Dr. R. Wobser GmbH & Co. KG, 97922 Lauda-Königshofen (DE)
(72) Erfinder: Firmbach, Peter, 97947 Grünsfeld (DE); Eller, Mathias, 97237 Altertheim (DE); Braun, Kristofer, 97980 Bad Mergentheim (DE); Gärtner, Rüdiger, 97941 Hochhausen (DE); Hefner, Patrick, 97249 Eisingen (DE)
(74) Vertreter: Zimmermann & Partner Patentanwälte mbB

(57) **Zusammenfassung**

Eine Vorrichtung zur extrakorporalen Temperierung von Patienten, ein Verfahren zum Betreiben eines externen Temperierkreislaufs für eine extrakorporale Apparatur, und die Verwendung eines externen Temperiersystems werden hierin beschrieben. Die Vorrichtung weist ein Hypothermiegerät und ein externes Temperiersystem zum Temperieren einer extrakorporalen Apparatur auf. Das externe Temperiersystem hat ein Leitungssystem (110) für ein Temperiermedium (157) und eine mit dem Leitungssystem (110) verbundene Befülleinrichtung (150) zum Befüllen und Entleeren des Temperiermediums in das Leitungssystem (110).

## Beschreibung

### Gebiet der Erfindung

Die Offenbarung betrifft eine Vorrichtung zur extrakorporalen Temperierung von Patienten, ein Verfahren zum Betreiben eines externen Temperierkreislaufs für eine extrakorporale Apparatur, und eine Verwendung eines externen Temperiersystems.

### Stand der Technik

Die Reinheit und Keimfreiheit von Kühlkreisläufen von Hypothermiegeräten ist insbesondere für eine Anwendung im Umfeld von bzw. im Zusammenhang mit medizinischen Operationen relevant. Aus dem Stand der Technik sind Hypothermiegeräte bekannt bei denen das Temperiermedium im Gerät in entsprechenden Tanks vorgehalten wird. Die Befüllung des Tanks erfolgt manuell. Während der Anwendung wird der Temperierkreis für die extrakorporale Apparatur, beispielsweise einem Oxygenator, aus diesen Vorratstank bedient. Das gesamte Wasser im Tank muss vor der Operation erst eingefüllt und auf die benötigte Temperatur gebracht werden. Dies erfordert eine längere Vorbereitungszeit.

Eine weitere aus dem Stand der Technik bekannte Variante ist es, Wasser in einem Reservoir zu gefrieren und dann nach Bedarf abzutauen und das kalte Wasser dem Temperierkreis beizumischen. Nachteilig ist, dass die gespeicherte Energie und damit die Anwendungsdauer begrenzt ist, die Vorbereitungszeit zur Ausbildung des Eisblockes entsprechend lange ist. Oftmals sind diese Geräte dauerhaft eingeschaltet, um den Eisblock vorzuhalten, was einen hohen Energiebedarf darstellt, um sicherzustellen, dass die Geräte kurzfristig einsatzbereit sind.

Eine restlose Entleerung des Temperierkreises und aller wasserführenden Teilen ist bei den aus dem Stand der Technik bekannten Hypothermiegeräten nicht möglich.

Insgesamt weist der Stand der Technik Nachteile in Bezug auf die Energieeffizienz, die Keimbelastung, und die Vorbereitungszeit sowie den Personalaufwand zum Bedienen von Vorrichtungen zur extrakorporalen Temperierung von Patienten auf.

### Offenbarung der Erfindung

Aufgabe der Erfindung ist es, eine Vorrichtung zur extrakorporalen Temperierung von Patienten anzugeben, welche gegenüber dem Stand der Technik verbessert ist. Insbesondere soll eine Vorrichtung zur extrakorporalen Temperierung von Patienten angegeben werden, welche eine automatische Befüllung und/oder Entleerung eines externen Temperierkreislaufs ermöglicht oder zu mindestens den zeitlichen Aufwand für einen Benutzer zum Befüllen und/oder Entleeren des externen Temperierkreislaufs reduziert.

Die Aufgabe wird mit einer Vorrichtung zur extrakorporalen Temperierung von Patienten nach Anspruch 1 sowie einem Verfahren zum Betreiben eines externen Temperierkreislaufs für eine extrakorporale Apparatur, und einer Verwendung eines externen Temperiersystems gemäß den nebengeordneten Ansprüchen gelöst.

Ein erster Aspekt betrifft eine Vorrichtung zur extrakorporalen Temperierung von Patienten. Die Vorrichtung weist ein Hypothermiegerät und ein externes Temperiersystem zum Temperieren einer extrakorporalen Apparatur auf. Das externe Temperiersystem weist ein Leitungssystem für ein Temperiermedium und eine mit dem Leitungssystem verbundene Befülleinrichtung zum Befüllen und Entleeren des Temperiermediums in das Leitungssystem auf. Die Befülleinrichtung weist eine Pumpe zum Fördern des Temperiermediums in das Leitungssystem oder aus dem Leitungssystem auf. Das Leitungssystem ist lösbar mit dem Hypothermiegerät verbunden und das Leitungssystem ist eingerichtet zum lösbaren Verbinden mit der extrakorporalen Apparatur. Das Hypothermiegerät ist dazu eingerichtet, ein in dem Leitungssystem befindliches Temperiermedium zu temperieren. Ferner ist die Befülleinrichtung und/oder das Leitungssystem, bevorzugt das externe Temperiersystem, frei von manuell betätigbaren Ventilen.

Unter dem "externen Temperiersystem" ist bevorzugt zu verstehen, dass das Temperiersystem vollständig extern in Bezug auf das Hypothermiegerät angeordnet ist, d.h. keine der Komponenten des externen Temperiersystems ist innerhalb des Hypothermiegeräts angeordnet. Insbesondere sind alle Komponenten des externen Temperiersystems außerhalb der Seitenwände oder der oberen Abdeckung angeordnet.

Die extrakorporale Apparatur ist bevorzugt eine extrakorporale Organersatzapparatur, insbesondere ein Oxygenator oder ein extrakorporales Membranoxygenierungs-Gerät.

Unter einem "manuell betätigbarem Ventil" ist ein Ventil zu verstehen, dass von einem Benutzer manuell bedient werden kann.

Die hierin offenbarte Vorrichtung ermöglicht eine Befüllung und Entleerung des Leitungssystems mit geringem Aufwand für einen Benutzer oder sogar ohne, dass ein Benutzer Komponenten des Temperiersystems betätigen muss. Gemäß einer bevorzugten Ausführungsform ist es vorgesehen, dass alle fluidfördernden Komponenten als auch alle Ventile der Befülleinrichtung und des Leitungssystems, bevorzugt das externe Temperiersystem, nicht manuell betätigbar sind. Hierdurch wird die Vorbereitungszeit reduziert bis die extrakorporale Apparatur in Betrieb genommen werden kann, und hilft gerade in Notfällen den Patienten schneller zu temperieren. Eine schnellere Rückführung des Temperiermediums nach Beendigung der Anwendung wird ebenfalls hierdurch ermöglicht. Ferner können das Leitungssystem, bzw. der externe Temperierkreislauf, und alle fluidfördernden Komponenten vollständig oder restlos entleert werden, ohne dass ein Benutzer Komponenten des Temperiersystems betätigen muss.

In einer bevorzugten Ausführungsform ist die Vorrichtung dazu eingerichtet ist, das Leitungssystem automatisch zu Befüllen oder zu Entleeren. Hierbei kann die Pumpe der Befülleinrichtung dazu eingerichtet sein das Leitungssystem automatisch zu Befüllen oder zu Entleeren. Unter automatisch ist bevorzugt zu verstehen, dass ein Befüll/Entleer-Vorgang, nach Aufforderung durch den Benutzer, vollständig ausgeführt wird, ohne dass der Benutzer mit der Vorrichtung interagieren muss, insbesondere ohne, dass der Benutzer Ventile der Befülleinrichtung betätigen muss.

Die Vorrichtung kann eine Kontrolleinheit aufweisen. Die Kontrolleinheit kann dazu eingerichtet sein, die Operation der Vorrichtung, insbesondere des Hypothermiegeräts und des externes Temperiersystems, zu kontrollieren.

Die Kontrolleinheit kann dazu eingerichtet sein eine Aufforderung oder einen Hinweis von dem Benutzer zu empfangen ein Befüllen oder Entleeren zu initiieren. Die Kontrolleinheit kann ferner dazu eingerichtet sein die Pumpe der Befülleinrichtung anzusteuern zum Ausführen eines Befüll/Entleer-Vorgangs.

Die Befülleinrichtung weist typischerweise ein Reservoir (oder einen Speicher) zum Bereitstellen des Temperiermediums für das externe Temperiersystem auf. Das Reservoir kann beispielsweise ein Beutel aus Folienmaterial oder eine Flasche oder ein sonstiges Behältnis für eine Flüssigkeit sein. In dem als Reservoir ein Beutel aus Folienmaterial verwendet wird, herrscht vorteilhafterweise in der Befülleinrichtung, unter Vernachlässigung von Höhenunterschieden, der atmosphärische Druck.

Das Temperiermedium ist typischerweise eine sterile Flüssigkeit, bevorzugt steriles endotoxinfreies Wasser.

Die Befülleinrichtung kann dazu eingerichtet sein, das Leitungssystem mit dem Temperiermedium aus dem Reservoir zu befüllen und/oder das Temperiermedium aus dem Leitungssystem in das Reservoir zu entleeren.

Ferner weist die Befülleinrichtung typischerweise eine Befüllleitung auf. Die Befüllleitung kann eingerichtet sein zum Befüllen des Leitungssystems. Die Befüllleitung kann das Reservoir mit dem Leitungssystem fluidisch verbinden. Ein erstes Ende der Befüllleitung kann mit dem Reservoir verbunden sein und/oder ein zweites Ende der Befüllleitung kann mit dem Leitungssystem, insbesondere einem Sekundärkörper des Leitungssystems, verbunden sein. Das externe Temperiersystem kann eine Anschlussstelle aufweisen, welche die Befülleinrichtung mit dem Leitungssystem, insbesondere dem Sekundärkörper, verbindet. Typischerweise weist das Leitungssystem die Anschlussstelle auf. Beispielsweise kann die Anschlussstelle ein Dreiwegesystem sein. Hierbei kann das Dreiwegesystem die Befüllleitung und einen Zulauf von einer Rücklaufleitung des Leitungssystems mit dem Sekundärkörper verbinden.

In manchen Ausgestaltungsformen weist die Befülleinrichtung ferner eine Ablaufleitung auf. Die Ablaufleitung kann das Reservoir mit dem Leitungssystem fluidisch verbinden. Ein erstes Ende der Ablaufleitung kann mit dem Reservoir verbunden sein und/oder ein zweites Ende der Ablaufleitung kann mit dem Leitungssystem, insbesondere einem Sekundärkörper des Leitungssystems, verbunden sein. Die Ablaufleitung kann auch als eine Überlaufleitung angesehen werden. Die Ablaufleitung ist dazu eingerichtet Temperiermedium und/oder Luft während des Betriebes und/oder beim Befüllen aus dem Temperierkreis zurück ins Reservoir zu bringen. Beim Entleeren ermöglicht die Ablaufleitung, dass Luft aus dem Reservoir in das Leitungssystem strömt. Ein Ablaufanschluss des Leitungssystems an die Ablaufleitung kann an einem höchsten Punkt des Sekundärkörpers, insbesondere des Leitungssystems, angeordnet ist. Ferner kann ein Ablaufanschluss der Ablaufleitung an das Reservoir an einem höchsten Punkt des Reservoirs angeordnet sein. Hierdurch kann das Leitungssystem entlüftet werden und verhindert werden, dass sich ein Überdruck in dem Temperiersystem aufbaut. Für den Fall, dass sich Luft in dem Leitungssystem befindet oder sammelt, wird hierdurch vorteilhafterweise ermöglicht, dass die Luft durch das Temperiermedium über die Ablaufleitung verdrängt wird, und (unter Vernachlässigung von Höhenunterschieden und Strömungswiderständen) ein atmosphärischer Druck im Sekundärkörper bei allen Betriebszuständen ermöglicht wird.

Die Pumpe zum Fördern des Temperiermediums in das Leitungssystem oder aus dem Leitungssystem kann in der Befüllleitung angeordnet sein. Typischerweise ist die Pumpe der Befülleinrichtung eine Peristaltikpumpe, beispielsweise eine selbstschließende Peristaltikpumpe. Die Pumpe kann dazu eingerichtet sein einen Fluidstrom in beide Richtungen zu erzeugen. Insbesondere kann die Pumpe dazu eingerichtet sein zum Entleeren des Leitungssystem eine Drehrichtung des Fluidflusses umzukehren. Ferner kann die Pumpe drehzahlgeregelt sein um unterschiedliche Förderströme zu erzeugen. Die Pumpe kann, in einem Ruhemodus oder bei Stillstand, sperrend sein, und damit insbesondere einen Fluss des Temperiermediums von dem Leitungssystem entlang der Befüllleitung sperren.

Ferner kann die Befülleinrichtung einen oder mehrerer Sensoren aufweisen. Der Sensor kann beispielsweise dazu eingerichtet sein einen Fluss des Temperiermediums, einen Füllstand des Temperiermediums, oder eine Luftkonzentration zu bestimmen. In einer Ausgestaltungsform ist der Sensor dazu eingerichtet einen Fluss des Temperiermediums in der Befüllleitung zu bestimmen. Der Sensor kann in der Befüllleitung angeordnet sein, insbesondere stromabwärts von der Pumpe. In einer anderen Ausgestaltungsform ist der Sensor ein Füllstandssensor, welcher dazu eingerichtet ist einen Füllstand des Temperiermediums in der Befüllleitung zu bestimmen. Dies erlaubt es Rückschlüsse auf einen Füllstand des Reservoirs zu ziehen.

Ferner kann die Vorrichtung dazu eingerichtet sein das Befüllen oder Entleeren des Leitungssystem anzuhalten falls ein Fluss oder ein Füllstand in der Befülleinrichtung unterhalb eines vorbestimmten Schwellenwert ist. Die Kontrolleinheit kann zusätzlich oder alternativ eingerichtet sein ein Alarmsignal auszugeben falls der Fluss oder der Füllstand in der Befülleinrichtung unterhalb des vorbestimmten Schwellenwert ist.

Das Reservoir kann ferner einen Befüllanschluss aufweisen zum Befüllen des Reservoirs mit Temperiermedium. Typischerweise wird das Reservoir lediglich ein Mal aufgefüllt, kann aber im Bedarfsfall bei einem Mangel an Temperiermedium über den Befüllanschluss nachgefüllt werden.

Das Reservoir kann einen Hydrophobfilter aufweisen, welcher einen Druckausgleich des Reservoirs mit der Umgebung ermöglicht. Der Hydrophobfilter verhindert sowohl, dass Luft mit Keimen in das Reservoir eindringen kann als auch Luft mit Keimen aus dem geschlossenen Temperiersystem/Reservoir austreten kann. Das System ist somit geschlossen, und Umgebungsluft bzw. Keime können weder eindringen noch entweichen.

Typischerweise enthält das Temperiersystem, insbesondere die Befülleinrichtung und das Leitungssystem eines oder mehrerer Ventile. Die Kontrolleinheit kann dazu eingerichtet sein jedes der einen oder mehreren Ventile zu betätigen, insbesondere zu öffnen oder schließen. Bei einem nicht manuell betätigbarem Ventil der Befülleinrichtung kann es sich auch um ein Rückschlagventil handeln.

Das externe Temperiersystem weist bevorzugt ein nicht-manuell betätigbares Ventil auf welches dazu konfiguriert ist einen Fluss des Temperiermediums zwischen der Befülleinrichtung und dem Leitungssystem einzustellen. Das nicht-manuell betätigbare Ventil kann dazu eingerichtet sein beim Befüllen zu öffnen, sodass Temperiermedium in das Leitungssystem gepumpt werden kann, und/oder beim Entleeren zu sperren. Typischerweise weist das Leitungssystem das nicht-manuell betätigbare Ventil auf. Das nicht-manuell betätigbare Ventil kann in die Anschlussstelle integriert sein.

Gemäß einer Ausführungsform ist die Vorrichtung, insbesondere die Kontrolleinheit dazu eingerichtet, zum Befüllen oder Entleeren des Leitungssystems, das Temperiermedium mittels der Pumpe der Befülleinrichtung für eine vorbestimmte Zeit zu fördern. Die Kontrolleinheit kann dazu eingereicht sein das Befüllen oder Entleeren auszuführen nachdem eine Aufforderung oder einen Hinweis von dem Benutzer empfangen wurde. Beispielsweise kann die vorbestimmte Zeit etwa 3 Minuten sein. Die Kontrolleinheit kann dazu eingerichtet sein, nach Ablauf der vorbestimmten Zeit, eine Förderleistung, beispielsweise eine Drehzahl, der Pumpe zu reduzieren oder in einen normalen Betriebsmodus zu wechseln. Die Kontrolleinheit kann ferner dazu eingerichtet sein, nach der vorbestimmten Zeit alle Pumpen zu stoppen und den Entleerungsvorgang zu beenden. In einem normalen Betriebsmodus kann die Befülleinrichtung weiterhin dazu eingerichtet sein Temperiermedium zwischen Befülleinrichtung und Leitungssystem zu fördern, um eine stetige Zirkulation des gesamten Temperiermediums zu erreichen.

Das Leitungssystem kann eine Vorlaufleitung und die Rücklaufleitung aufweisen. Die Vorlaufleitung und die Rücklaufleitung sind jeweils verbindbar mit der extrakorporalen Apparatur zum Ausbilden eines externen Temperierkreislaufs.

Das Leitungssystem kann ferner einen Vorlaufanschluss und einen Rücklaufanschluss aufweisen. Der Vorlaufanschluss und der Rücklaufanschluss des externen Temperiersystems können jeweils verbindbar mit der extrakorporalen Apparatur sein. Hierdurch wird die Ausbildung eines externen Temperierkreislaufs ermöglicht.

Gemäß einer Ausführungsform weist das Leitungssystem ferner eine Bypassleitung auf zum Ausbilden einer Fluidverbindung zwischen der Vorlaufleitung und der Rücklaufleitung. Die Bypassleitung ist bevorzugt mit der Vorlaufleitung im Bereich eines stromabwärtigen Endabschnitts der Vorlaufleitung, insbesondere in der Nähe des Vorlaufanschlusses, verbunden. Die Bypassleitung ist bevorzugt mit der Rücklaufleitung im Bereich eines stromaufwärtigen Endabschnitts der Rücklaufleitung, insbesondere in der Nähe des Rücklaufanschlusses, verbunden. Gemäß dieser Ausführungsform ist die Vorrichtung dazu eingerichtet das Leitungssystem über die Bypassleitung zu befüllen oder zu entleeren, ohne dass das externe Temperiersystem mit der extrakorporalen Apparatur verbunden sein muss oder verbunden ist. Die Vorrichtung kann ferner dazu eingerichtet sein das Temperiermedium auf eine vorbestimmte Temperatur zu temperieren vor Anschluss des Leitungssystems an die extrakorporale Apparatur und/oder ohne dass das externe Temperiersystem mit der extrakorporalen Apparatur verbunden sein muss oder verbunden ist. Das Temperiermedium kann dabei von der Vorlaufleitung über den Bypass in die Rücklaufleitung fließen. Dies ermöglicht es den externen Temperierkreislauf und die extrakorporale Apparatur schneller in Betrieb zu nehmen, da bereits bei Anschluss der extrakorporalen Apparatur ein betriebsfähiger und vortemperierter externer Temperierkreislauf zur Verfügung steht. Hierdurch wird die Vorbereitungszeit reduziert bis die extrakorporale Apparatur in Betrieb genommen werden kann, und hilft gerade in Notfällen den Patienten schneller zu temperieren. Eine schnellere Rückführung des Temperiermediums nach Beendigung der Anwendung wird hierdurch ebenfalls ermöglicht.

In einer Ausführungsform weisen die Vorlaufleitung und die Rücklaufleitung des Leitungssystems jeweils eine selbstschließende Kupplung auf zum Anschließen an die extrakorporale Apparatur. Die selbstschließende Kupplung verhindert ein Austreten des Temperiermediums, wenn die jeweilige Leitung nicht mit der extrakorporalen Apparatur verbunden ist bzw. schließt selbstständig, wenn eine Leitung von der extrakorporalen Apparatur abgekuppelt wird. Die selbstschließende Kupplung ermöglicht einen Fluss zwischen der Leitung und der extrakorporalen Apparatur, wenn die Leitung mit der extrakorporalen Apparatur gekuppelt ist. Somit weist die Vorrichtung im Bereich des Vorlaufanschlusses und des Rücklaufanschlusses, und insgesamt im gesamtem Leitungssystem, bevorzugt keine manuell betätigbaren Ventile auf. Hierdurch kann sowohl das Befüllen und/oder Entleeren, als auch das Ausbilden und das Vortemperieren des externen Temperierkreislaufs automatisch ermöglicht werden, bzw. ermöglicht werden ohne dass ein Benutzer Komponenten des externen Temperiersystems betätigen muss. Ferner wird eine kürzere Abrüstzeit ohne Wasserverlust beim Abkoppeln der extrakorporalen Apparatur ebenfalls ermöglicht. Im Fall von mehreren vorhandenen Kreisläufen ermöglicht die hierin offenbarte Vorrichtung weiterhin, dass innerhalb kurzer Zeit die extrakorporale Apparatur je nach Bedarf gewechselt werden kann.

Das Leitungssystem kann ferner den Sekundärkörper aufweisen. Der Sekundärkörper ist dazu eingerichtet mit einem externen Primärkörper des Hypothermiegeräts gekoppelt zu werden.

Unter dem "externen Primärkörper" des Hypo-/Hyperthermiegeräts ist zu verstehen, dass der Primärkörper nicht innerhalb des Hypothermiegeräts angeordnet ist. Das Hypothermiegerät kann eine oder mehrere Seitenwände oder obere und untere Abdeckungen aufweisen. Der Primärkörper ist entweder außerhalb der Seitenwände und der Abdeckungen angeordnet oder der Primärkörper ist in einer der Seitenwände oder der oberen Abdeckung integriert. Mit anderen Worten kann der externe Primärkörper als ein von außen zugänglicher Primärkörper betrachtet werden.

Durch das Kuppeln des externen Primärkörpers des Hypothermiegeräts zusammen mit dem Sekundärkörper des externen Temperiersystems wird ein Wärmetauscher ausgebildet. Insbesondere ist das Hypothermiegerät dazu eingerichtet Wärme- oder Kühlleistung von dem Primärkörper an den Sekundärkörper des externen Temperiersystems zu übertragen. Der externe Primärkörper kann Mittel aufweisen um das durch den Sekundärkörper strömende Temperiermedium zu temperieren, d.h. zu kühlen oder zu erhitzen. Beispielsweise können die Mittel ein Heizelement und/oder ein Verdampfer sein.

Ein stromaufwärtiger Endabschnitt der Vorlaufleitung und/oder ein stromabwärtiger Endabschnitt der Rücklaufleitung können jeweils mit dem Sekundärkörper verbunden sein, mechanisch und/oder fluidisch.

In einer Ausführungsform weist das externe Temperiersystem, insbesondere das Leitungssystem, eine Ultraviolett(UV)-Quelle zum Desinfizieren des Temperiermediums auf. Durch die Bestrahlung mit ultraviolettem Licht im Temperiersystem kann eine kontinuierliche Sterilisation des Temperiermediums erreicht werden. Die Nutzung von ultraviolettem Licht reduziert oder vermeidet den Einsatz chemischer Reinigungsmittel und kann, insbesondere bei Nutzung einer geeigneten ultravioletten Wellenlänge, ein Auftreten des Mycobacterium chimaera verhindern.

Ultraviolette Strahlung umfasst typischerweise Licht im UV-A, UV-B und UV-C Spektralbereich. Insbesondere umfasst der UV-A-Spektralbereich Licht mit Wellenlängen von 315 nm bis 400 nm, der UV-B-Spektralbereich Licht mit Wellenlängen von 280 nm bis 315 nm und der UV-C-Spektralbereich Licht mit Wellenlängen von 200 nm bis 280 nm. In bevorzugten Ausführungsformen erzeugt die UV-Quelle ultraviolette Strahlung im UV-C-Spektralbereich, insbesondere bei einer Wellenlänge von 265 nm. Typischerweise wird eine Emissionswellenlänge der UV-Quelle von im Wesentlichen 265 nm mit einer besonders bevorzugten Desinfektionsleistung assoziiert. Insbesondere reduziert ultraviolettes Licht mit einer Wellenlänge von im Wesentlichen 265 nm die Anzahl von Viren, Bakterien und chlorbeständigen pathogenen Keimen besonders stark.

Die UV Quelle kann beispielsweise in der Vorlaufleitung angeordnet sein. Die Vorrichtung kann dazu eingerichtet sein kontinuierlich, z.B. sowohl während dem Befüllvorgang als auch in dem normalen Betriebsmodus, Temperiermedium von der Befülleinrichtung durch das Leitungssystem zu zirkulieren. Somit wird das Reservoir dauerhaft gespült und das gesamte im externen Temperiersystem befindliche Temperiermedium wird regelmäßig bestrahlt. Hierdurch kann eine Keimbelastung des gesamten im externen Temperiersystem befindlichen Temperiermediums reduziert werden.

In einer Ausführungsform weist das Leitungssystem einen in der Vorlaufleitung angeordneten (ersten) Temperatursensor auf. Hierdurch wird eine genaue Messung der (Vorlauf-)Temperatur des Temperiermediums an einer Stelle ermöglicht kurz bevor das Temperiermedium mit einer Körperflüssigkeit in der extrakorporalen Apparatur in thermischer Wechselwirkung tritt. Ferner kann das Leitungssystem zusätzlich einen in der Rücklaufleitung angeordneten (zweiten) Temperatursensor aufweisen zum Messen einer (Rücklauf-)Temperatur. Die Kontrolleinheit kann dazu eingerichtet sein, eine Heizleistung des externen Primärkörpers des Hypothermiegeräts zu kontrollieren in Abhängigkeit der vom (ersten und/oder zweiten) Temperatursensor ermittelten Fluidtemperatur. Der erste Temperatursensor ist bevorzugt stromabwärts von der UV Quelle, falls vorhanden, in der Vorlaufleitung angeordnet.

Typischerweise weist das Leitungssystem ferner mindestens eine Pumpe auf zum Fördern des Temperiermediums innerhalb des externen Temperierkreislaufs. Die Kontrolleinheit kann dazu eingereicht sein die mindestens eine Pumpe des Leitungssystems anzusteuern zum Ausführen eines Befüll/Entleer-Vorgangs.

Gemäß einer Ausführungsform weist das Leitungssystem eine in der Vorlaufleitung angeordnete Vorlaufpumpe und eine in der Rücklaufleitung angeordnete Rücklaufpumpe auf. Die Vorlaufpumpe und die Rücklaufpumpe können jeweils eine einstellbare Förderleistung aufweisen. Die Kontrolleinheit kann dazu eingerichtet sein, die Vorlaufpumpe und die Rücklaufpumpe einzeln anzusteuern und deren Förderleistung jeweils individuell einzustellen. Die Vorlaufpumpe kann dazu eingerichtet sein, einen individuell einstellbaren Über- oder Unterdruck in der Vorlaufleitung auszubilden, und/oder die Rücklaufpumpe kann dazu eingerichtet sein, einen individuell einstellbaren Über- oder Unterdruck in der Rücklaufleitung auszubilden. Beispielsweise kann die Kontrolleinheit dazu eingerichtet sein die Vorlaufpumpe so anzusteuern, dass ein Überdruck in der Vorlaufleitung ausgebildet wird und/oder die Rücklaufpumpe so anzusteuern, dass ein Unterdruck in der Rücklaufleitung ausgebildet wird. Die (Vor-/Rücklauf-)pumpen können somit individuelle Über / Unterdrücke an die extrakorporale Apparatur bereitstellen. Vorteilhafterweise kann durch Verwendung von zwei getrennt drehzahlregelbaren Pumpen an der extrakorporalen Apparatur ein individueller Überdruck am Vorlauf und ein individueller Unterdruck am Rücklauf realisiert werden, wodurch die bestimmungsgemäße Verwendung einer großen Bandbreite von Fabrikaten von extrakorporalen Apparaturen ermöglicht wird. Ferner wird durch die Verwendung von zwei getrennt drehzahlregelbaren Pumpen es ermöglicht den Sekundärkörper (unter Vernachlässigung von Höhenunterschieden und Strömungswiderständen) drucklos zu betreiben, wodurch eine besonders hohe Sicherheit bei der Abdichtung zwischen Primärkörper und Sekundärkörper erreicht wird.

Gemäß einer Ausführungsform weist das externe Temperiersystem ein Umschaltventil auf. Das Umschaltventil ist in dem externen Temperiersystem angeordnet. Das Umschaltventil weist einen ersten und einen zweiten Zustand auf.

Das Umschaltventil ist typischerweise sowohl mit der Befüllleitung als auch mit der Vorlauf- und/oder Rücklaufleitung in Fluidverbindung eingebunden. In anderen Worten definiert das Umschaltventil typischerweise einen Abschnitt der Befüllleitung und der Vorlauf- und/oder Rücklaufleitung. Bevorzugt handelt es sich bei dem Umschaltventil um ein 4-Wege Umschaltventil.

In dem ersten Zustand des Umschaltventils ist die Befülleinrichtung, insbesondere die Befüllleitung, mit der Vorlaufleitung in Fluidverbindung eingebunden. Ferner kann im ersten Zustand die Rücklaufleitung mit dem Sekundärkörper in Fluidverbindung eingebunden sein.

In dem zweiten Zustand des Umschaltventils ist die Befülleinrichtung, insbesondere die Befüllleitung, mit der Rücklaufleitung in Fluidverbindung eingebunden. Ferner kann im zweiten Zustand der Sekundärkörper mit der Vorlaufleitung in Fluidverbindung eingebunden sein.

Der erste Zustand ermöglicht einen besonders schnellen und simplen Befüllvorgang. Das Temperiermedium kann mit Schwerkraft Antrieb selbstständig aus dem Reservoir in das Leitungssystem fließen. Gleichzeitig wird auch eine Pumpe, bevorzugt die Vorlaufpumpe, vorgefüllt. Anschließend können die Vorlauf- und die Rücklaufpumpen in Betrieb genommen werden. Restliche im System verbliebene Luft entweicht während dem normalen Betrieb kontinuierlich durch die Ablaufleitung in das Reservoir und während das Reservoir Temperiermedium ins System nachspeist. Somit funktioniert das Füllen des Temperiersystems mit Temperiermedium sehr einfach durch Schwerkraft Antrieb. Mit dem anschließenden Zuschalten der Pumpen beginnt ein kontinuierlicher Entlüftungsprozess während dem ganzen Betrieb.

Der zweite Zustand ermöglicht einen besonders schnellen und simplen Entleervorgang. Insbesondere ist zur Entleerung des Temperiermediums lediglich ein Umschalten des Umschaltventiles vom ersten in den zweiten Zustand erforderlich. Die Entleerung des Leitungssystems kann erfolgen in dem bei laufende Vorlauf- und Rücklaufpumpe das Umschaltventil in den zweiten Zustand umgeschaltet wird. Dadurch wird die Strömung durch das Reservoir umgekehrt. Die Vorlauf- und die Rücklaufpumpe entleeren beginnend mit der Ablaufleitung den Sekundärkörper, die Vorlaufpumpe, die Vorlauf- und Rücklaufleitungen hin und zurück von der extrakorporalen Apparatur bis sich die Rücklaufpumpe selbst entleert. In diesem Zustand kann die extrakorporale Apparatur, beispielsweise ein Oxygenator, problemlos gewechselt werden. Ferner kann das Hypothermiegerät vollständig vom externen Temperierkreis getrennt werden ohne jegliche verbleibende Restfeuchtigkeit. Nach Entfernen des Medienkreises ist die thermische Kontaktfläche des Primärkörpers sehr leicht zugänglich und reinigbar.

Gemäß einem allgemeinen Aspekt der vorliegenden Offenbarung ist es vorgesehen, dass das externe Temperiersystem bevorzugt als Einwegtemperiersystem verwendet wird und insbesondere nicht gereinigt wird. Bevorzugterweise werden alle Komponenten des externen Temperiersystems nach Verwendung entsorgt.

"Einwegtemperiersystem" kann eine einmalige Nutzung bedeuten. "Einwegtemperiersystem" kann aber auch eine mehrmalige Nutzung bedeuten, wobei nach einem vordefinierten maximalen Nutzungszeitraum das als Einwegtemperiersystem ausgebildete externe Temperiersystem durch ein neues, ebenfalls als Einwegtemperiersystem ausgebildetes, externes Temperiersystem ersetzt wird. Typischerweise entspricht der vordefinierte maximale Nutzungszeitraum einer Anwendungszeit bei der das Temperiermedium vordefinierte maximale Hygienegrenzwerte einhält. In einer Ausführungsform beträgt der vordefinierte maximale Nutzungszeitraum höchstens 35 Tage, bevorzugt höchstens 14 Tage oder mindestens eine Nutzung, bevorzugt mindestens 2 Tage. Unabhängig davon ob das externe Temperiersystem zur einmaligen Nutzung oder zur mehrmaligen Nutzung verwendet wird, kann im Anschluss daran das externe Temperiersystem durch ein neues externes Temperiersystem ersetzt werden. Der Ersatz des externen Temperiersystems durch ein neues externes Temperiersystem nach einer bestimmten Anwendungsdauer gewährleistet einen sicheren und hygienischen Betrieb des externen Temperierkreislaufs für die extrakorporale Apparatur, insbesondere über die komplette Lebensdauer des Hypothermiegeräts. Mit der Erfindung kann eine längerer Nutzungszeitraum für das Temperiermedium, exemplarisch bis zu 35 Tage, erreicht werden ohne einen Wechsel des Temperiermediums.

Gemäß einer alternativen Ausführungsform kann das externe Temperiersystem, und insbesondere die Befülleinrichtung manuell betätigbare Ventile aufweisen.

Ein zweiter Aspekt betrifft ein Verfahren zum Betreiben eines externen Temperierkreislaufs für eine extrakorporale Apparatur. Das Verfahren umfasst den Schritt i) Lösbares Verbinden eines Hypothermiegeräts, insbesondere dem Hypothermiegerät nach einem der hierin offenbarten Ausführungsformen, mit einem externen Temperiersystem zum Temperieren einer extrakorporalen Apparatur, insbesondere dem externen Temperiersystem nach einem der hierin offenbarten Ausführungsformen.

Das Verfahren umfasst den Schritt ii) Lösbares Verbinden des externen Temperiersystems mit der extrakorporalen Apparatur zum Ausbilden eines externen Temperierkreislaufs.

Das Verfahren umfasst ferner den Schritt iii) Automatisches Befüllen des externen Temperierkreislaufs mit einem Temperiermedium mittels einer Befülleinrichtung, ohne manuellen Betätigens eines Ventils des externen Temperiersystems.

Hierbei werden die Schritte ii) und iii) nach Schritt i) ausgeführt. Jedoch wird Schritt iii) bevorzugt auch vor Schritt ii) ausgeführt. In diesem Fall erfolgt die Befüllung des Leitungssystem mittels der Bypassleitung.

Das Verfahren kann ferner nach Schritt i) den Schritt iv) Befüllen der Befülleinrichtung, insbesondere des Reservoirs, mit dem Temperiermedium über den Befüllanschluss des Reservoirs. Schritt iv) erfolgt vor Schritt iii), und kann vor Schritt ii) erfolgen.

Schritt iii) kann ferner umfassen Fördern des Temperiermediums von dem Reservoir über die Befüllleitung in das Leitungssystem, insbesondere dem Sekundärkörper des Leitungssystems, mittels der Pumpe der Befülleinrichtung.

Das Verfahren kann ferner, im Anschluss an Schritt iii), und optional vor Schritt ii), den Schritt v) Temperieren des Temperiermediums mittels dem Hypothermiegerät und optional Bestrahlen des Temperiermediums mittels der UV Quelle umfassen.

Nach Beendigung einer Anwendung mit der extrakorporalen Apparatur kann das Verfahren ferner den Schritt vi) Entleeren des Temperiermediums aus dem Leitungssystem umfassen.

Ein dritter Aspekt betrifft eine Verwendung eines externen Temperiersystems nach einem der hierin offenbarten Ausführungsformen und/oder einem Hypothermiegerät nach einem der hierin offenbarten Ausführungsformen zum Befüllen und Entleeren des Temperiermediums in das Leitungssystem ohne dass Ventile des externen Temperiersystems manuell betätigt werden müssen.

### Kurze Beschreibung der Zeichnung

Nachfolgend wird die Erfindung anhand der beiliegenden Zeichnungen näher erläutert, wobei die Figuren zeigen:
- Fig. 1: eine Vorrichtung zur extrakorporalen Temperierung von Patienten gemäß Ausführungsformen der vorliegenden Offenbarung;
- Fig. 2: eine Vorrichtung zur extrakorporalen Temperierung von Patienten gemäß Ausführungsformen der vorliegenden Offenbarung;
- Fig. 3: eine Vorrichtung zur extrakorporalen Temperierung von Patienten gemäß Ausführungsformen der vorliegenden Offenbarung während einer Befüllung mit einem Temperiermedium;
- Fig. 4: eine Vorrichtung zur extrakorporalen Temperierung von Patienten gemäß Ausführungsformen der vorliegenden Offenbarung während einer Entleerung des Temperiermediums.

### Beschreibung von Ausführungsformen

Nachfolgend werden typische Ausführungsbeispiele anhand der Figuren beschrieben, wobei die Erfindung nicht auf die Ausführungsbeispiele beschränkt ist. Vielmehr wird der Umfang der Erfindung durch die Ansprüche bestimmt. Bei der Beschreibung der Ausführungsformen werden unter Umständen in verschiedenen Figuren und für verschiedene Ausführungsformen gleiche Bezugszeichen für gleiche oder ähnliche Teile verwendet, um die Beschreibung übersichtlicher zu gestalten. Dies bedeutet jedoch nicht, dass entsprechende Teile der Erfindung auf die in den Ausführungsformen dargestellten Varianten beschränkt sind. Teilweise werden Merkmale, welche bereits im Zusammenhang mit anderen Figuren beschrieben wurden, der Übersichtlichkeit halber nicht nochmals beschrieben. Teilweise sind Merkmale, welche in einer Figur mehrfach dargestellt sind, lediglich einmal mit Bezugszeichen gekennzeichnet.

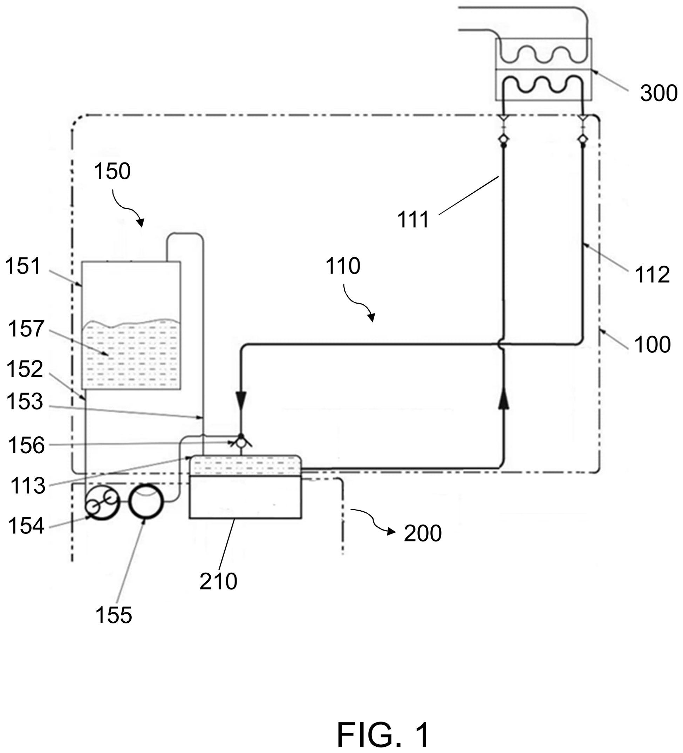

Fig. 1 zeigt eine Vorrichtung zur extrakorporalen Temperierung von Patienten. Die Vorrichtung weist ein Hypo-/Hyperthermiegerät 200 auf. Das Hypothermiegerät 200 weist einen externer Primärkörper 210 auf. Der Primärkörper hat ein Heizelement 201 und/oder einen Verdampfer 202 zum Temperieren, d.h. Heizen oder Kühlen, des Primärkörpers 210.

Die Vorrichtung weist ferner ein externes Temperiersystem 100 zum Temperieren einer extrakorporalen Apparatur 300 auf. Bei der extrakorporalen Apparatur 300 handelt es sich um einen Oxygenator.

Das externe Temperiersystem 100 weist ein Leitungssystem 110 und eine Befülleinrichtung 150 auf.

Das Leitungssystem 110 ist lösbar mit dem Hypothermiegerät 200 verbunden. Das Leitungssystem weist einen Sekundärkörper 113 auf, welcher mechanisch mit dem Hypothermiegerät 200 lösbar verbunden ist und thermisch mit dem externen Primärkörper 210 gekoppelt ist. Das Hypothermiegerät 200 kann durch die thermische Kopplung zwischen Primär- und Sekundärkörper 210, 113 die Temperatur von durch den Sekundärkörper 210 strömendem Fluid einstellen.

Das Leitungssystem 110 weist ferner eine Vorlaufleitung 111 und eine Rücklaufleitung 112, welche jeweils an einem Ende mit dem Sekundärkörper 113 verbunden sind und an einem anderen Ende mit der extrakorporalen Apparatur 300 lösbar verbunden werden können bzw. in Figur 1 mit der extrakorporalen Apparatur 300 lösbar verbunden sind.

Die Befülleinrichtung 150 ist eingerichtet zum Befüllen und Entleeren eines Temperiermediums 157 in das Leitungssystem 110. Die Befülleinrichtung 150 weist ein Reservoir 151 auf, welches einem Kunststoffbeutel entspricht und mit Temperiermedium 157 befüllt ist bzw. befüllbar ist.

Die Befülleinrichtung 150 weist ferner eine Befüllleitung 152 zum Befüllen des Leitungssystems 110 mit Temperiermedium 157 und eine Ablaufleitung 153 um Temperiermedium 157 und/oder Luft während des normalen Betriebsmodus (oder Temperierbetrieb) und/oder beim Befüllen aus dem Leitungssystem 110 zurück ins Reservoir 151 zu bringen.

Ferner weist die Befülleinrichtung 150 eine Peristaltikpumpe 154 oder Rollerpumpe auf um das Temperiermedium 157 von der Befülleinrichtung 150 hin zum Leitungssystem 110 zu fördern.

Die Befülleinrichtung 150 kann einen Sensor 155 aufweisen. Der in Figur 1 gezeigte Sensor 155 erlaubt es zu detektieren ob Temperiermedium durch die Befülleitung gepumpt wird. Kommt Luft in die Befülleitung, wird dies detektiert und das System reagiert entsprechend.

Die Befülleinrichtung 150, und in Figur 1 das gesamte externe Temperiersystem 100, sind frei von manuell betätigbaren Ventilen. Die Befüllleitung 152 ist mit dem Sekundärkörper 210 über eine Anschlussstelle 156 verbunden. Hier handelt es sich bei der Anschlussstelle 156 um ein Dreiwegesystem, bei dem die Befüllleitung 152 und ein Zulauf der Rücklaufleitung 112 des Leitungssystems 110 mit dem Sekundärkörper 113 verbunden sind. Im Bereich der Anschlussstelle 156 weist die Befülleinrichtung 150 ein Ventil auf, welches, beim Befüllen (oder Befüllbetrieb) durch die Perestaltikpumpe 154, geöffnet werden kann. Dadurch kann in dem gesamten Temperierkreis Temperiermedium 157 gebracht werden.

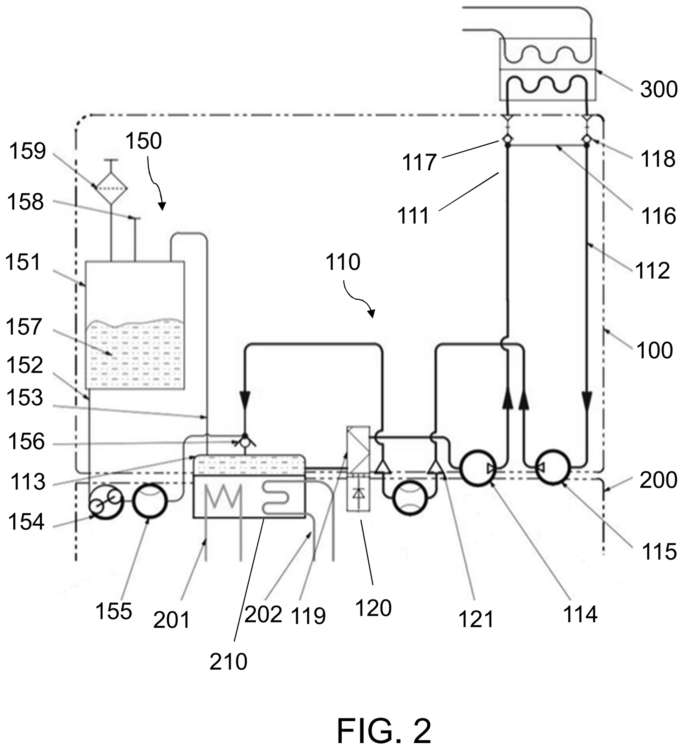

In einem normalen Betriebsmodus (oder Temperierbetrieb) kann das Ventil durch eine Pumpe im Leitungssystem 110 geöffnet werden (nicht in Figur 1 gezeigt; Figur 2 zeigt eine Pumpe im Leitungssystem). Der Kreislauf kann kontinuierlich zirkulieren.

Die Peristaltikpumpe 154 pumpt kontinuierlich Temperiermedium 157 aus dem Reservoir 151 in den Temperierkreis. Luftblasen im System werden sicher über die Ablaufleitung 153 abgeschieden, fehlendes Volumen ersetzt. Die hierin offenbarte Vorrichtung ermöglicht eine Befüllung und Entleerung des Leitungssystems mit geringem Aufwand für einen Benutzer oder sogar ohne, dass ein Benutzer Komponenten des Temperiersystems betätigen muss.

Fig. 2 zeigt eine Vorrichtung zur extrakorporalen Temperierung von Patienten gemäß einer weiteren Ausführungsform. Viele der in Figur 2 gezeigten Merkmale sind bereits im Zusammenhang mit Figur 1 erläutert worden. Für diese Merkmale wird auf die vorstehenden Erläuterungen verwiesen.

Die Befülleinrichtung 150 weist einen Befüllanschluss 158 auf zum Befüllen des Reservoirs 151 mit Temperiermedium 157.

Ferner weist die Befülleinrichtung 150 einen hydrophoben Filter 159 auf, welcher einen Druckausgleich des Reservoirs 151 mit der Umgebung ermöglicht und verhindert, dass Luft mit Keimen in das Reservoir 151 gelangen kann als auch Luft mit Keimen aus dem geschlossenen Reservoir 151 austreten kann.

Das Leitungssystem 110 weist zwei selbstschließende Kupplungen 117, 118 auf. Selbstschließende Kupplung 117 ist eingerichtet zum Kuppeln der Vorlaufleitung 111 mit der extrakorporalen Apparatur 300. Selbstschließende Kupplung 118 ist eingerichtet zum Kuppeln der Rücklaufleitung 112 mit der extrakorporalen Apparatur 300. Die selbstschließenden Kupplungen 117, 118 verhindern ein Austreten des Temperiermediums 157 wenn die jeweilige Leitung 111, 112 nicht mit der extrakorporalen Apparatur 300 verbunden ist bzw. schließen selbstständig wenn eine Leitung 111, 112 von der extrakorporalen Apparatur 300 abgekuppelt wird.

Ferner weist das Leitungssystem 110 eine Bypassleitung 116 auf, welche die Vorlaufleitung 111 und die Rücklaufleitung 112 fluidisch verbindet. Hiermit kann ein geschlossener Temperierkreislauf ausgebildet werden auch ohne, dass das Leitungssystem 110 mit dem Oxygenator 300 verbunden ist. Dies ermöglicht das Leitungssystem 110 vollständig mit dem Temperiermedium 157 zu durchströmen und auf die gewünschte Anwendungstemperatur zu bringen bevor das Leitungssystem 110 an die extrakorporalen Apparatur 300 angeschlossen wird.

Die Bypassleitung 116 ist hydraulisch so gestaltet, dass bei angekoppeltem Oxygenator 300 der größte Anteil des Temperiermediums 157 durch den Oxygenator 300 strömt. Beim Abkoppeln oder vor dem Ankoppeln kann aber ausreichend Temperiermedium durch die Bypassleitung 116 geführt werden um den Befüll und/ oder Entleervorgang durchzuführen.

Nach dem Befüllen des Leitungssystems 110 ist eine Vortemperierung des Temperiermediums über die Bypassleitung 116 möglich. Das Leitungssystem 110 weist eine UV Quelle 119 auf, welche in der Vorlaufleitung 111 angeordnet ist. Durch die Bestrahlung mit ultraviolettem Licht im Temperiersystem 100 kann eine kontinuierliche Sterilisation des Temperiermediums 157 erreicht werden.

Das Leitungssystem 110 weist eine in der Vorlaufleitung 111 angeordnete Vorlaufpumpe 114 und eine in der Rücklaufleitung 112 angeordnete Rücklaufpumpe 115 auf.

Ferner weist das Leitungssystem 110 einen in der Vorlaufleitung 111 angeordneten Temperatursensor 120 auf und einen in der Rücklaufleitung 112 angeordneten Temperatursensor 121 auf.

Das Temperiermedium 157 im Temperierkreis und das Temperiermedium im Reservoir 151 können kontinuierlich ausgetauscht werden. Dadurch wird ermöglicht, dass alles Temperiermedium 157 kontinuierlich an der UV Quelle 119 vorbeigeführt wird.

Temperiermedium 157 kann auch im "Wartezustand" vor der Anwendung, insbesondere bei noch nicht angekoppeltem Oxygenator 300, an der UV Quelle 119 vorbeigeführt werden. Eine Desinfektion des Temperiermediums 157 ist so auch vor oder nach der Anwendung möglich.

Beim Entleeren werden die Vorlaufpumpe 114 und die Rücklaufpumpe 115 des Temperierkreises gestoppt. Das Ventil schließt. Durch Umkehrung der Förderrichtung der Peristaltikpumpe kann das gesamte Leitungssystem 110 inklusive Sekundärkörper 113 über die Vorlaufleitung 111 entleert werden. Aufgrund der Verbindung des Sekundärkörpers 113 über die Ablaufleitung 153 mit dem Reservoir 151 kann das Temperiermedium in das Reservoir 151 gepumpt werden und es kann die benötigte Luft in den Temperierkreis nachströmen. Das System bleibt druckneutral.

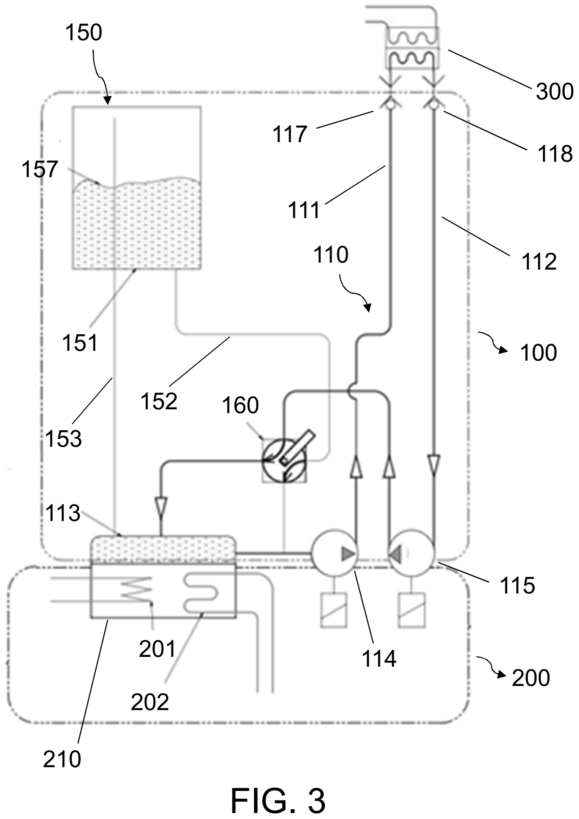

Fig. 3 zeigt eine Vorrichtung zur extrakorporalen Temperierung von Patienten gemäß einer weiteren Ausführungsform während einer Befüllung mit einem Temperiermedium 157 bzw. während einem normalen Betriebsmodus. Viele der in Figur 3 gezeigten Merkmale sind bereits im Zusammenhang mit Figur 1 erläutert worden. Für diese Merkmale wird auf die vorstehenden Erläuterungen verwiesen.

Das externe Temperiersystem 100 weist eine Anschlussstelle 160 mit einem Umschaltventil auf. Das Umschaltventil weist einen ersten und einen zweiten Zustand auf. Figur 3 zeigt das Umschaltventil im ersten Zustand. Bei dem Umschaltventil handelt es sich um ein 4-Wege Umschaltventil.

Das Umschaltventil ist mit der Befüllleitung 152 und mit dem Leitungssystem 110 in Fluidverbindung eingebunden. In dem ersten Zustand des Umschaltventils ist die Befüllleitung 152 mit der Vorlaufleitung 111 in Fluidverbindung eingebunden. Ferner ist im ersten Zustand die Rücklaufleitung 112 mit dem Sekundärkörper 113 in Fluidverbindung eingebunden.

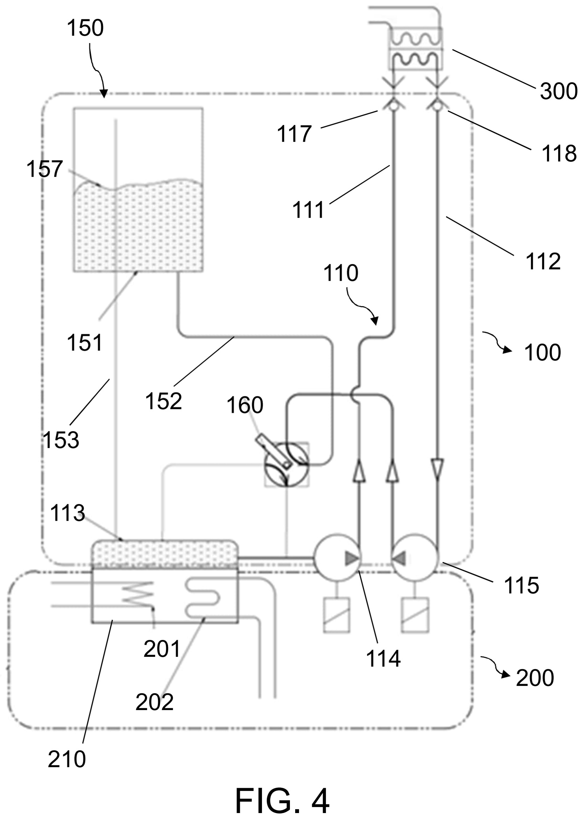

Figur 4 zeigt die Vorrichtung aus Figur 3 im zweiten Zustand. Das Umschaltventil 160 befindet sich im zweiten Zustand zur Entleerung des Temperiermediums 157 aus dem Leitungssystem 110 in das Reservoir 151.

In dem zweiten Zustand des Umschaltventils ist die Befüllleitung 152 mit der Rücklaufleitung 112 in Fluidverbindung eingebunden. Ferner ist im zweiten Zustand der Sekundärkörper 113 mit der Vorlaufleitung 111 in Fluidverbindung eingebunden.

Diese Ausführungsform ermöglicht einen besonders schnellen und simplen Befüll- als auch Entleervorgang. Ferner kann zwischen den beiden Zuständen geschaltet werden durch Betätigen lediglich eines Ventils. Das Temperiermedium kann zum Befüllen im ersten Zustand mit Schwerkraft Antrieb selbstständig aus dem Reservoir in das Leitungssystem fließen. Durch Umschalten in den zweiten Zustand wird die Strömung durch das Reservoir umgekehrt. Die Vorlauf- und die Rücklaufpumpe entleeren beginnend mit der Ablaufleitung den Sekundärkörper, die Vorlaufpumpe, die Vorlauf- und Rücklaufleitungen hin und zurück von der extrakorporalen Apparatur bis sich die Rücklaufpumpe selbst entleert. In diesem Zustand kann die extrakorporale Apparatur, beispielsweise ein Oxygenator, problemlos gewechselt werden. Ferner kann das Hypothermiegerät vollständig vom externen Temperierkreis getrennt werden ohne jegliche verbleibende Restfeuchtigkeit. Nach Entfernen des Medienkreises ist die thermische Kontaktfläche sehr leicht zugänglich und reinigbar.

### Bezugszeichenliste

- 100: externes Temperiersystem
- 110: Leitungssystem
- 111: Vorlaufleitung
- 112: Rücklaufleitung
- 113: Sekundärkörper
- 114: Vorlaufpumpe
- 115: Rücklaufpumpe
- 116: Bypassleitung
- 117, 118: selbstschließende Kupplung
- 119: UV-Quelle
- 120: Temperatursensor
- 121: Temperatursensor
- 150: Befülleinrichtung
- 151: Reservoir
- 152: Befüllleitung
- 153: Ablaufleitung
- 154: Pumpe
- 155: Sensor
- 156: Anschlussstelle
- 157: Temperiermedium
- 158: Befüllanschluss
- 159: Hydrophober Filter
- 160: Anschlussstelle mit Umschaltventil
- 200: Hypothermiegerät
- 201: Heizelement
- 202: Verdampfer
- 210: externer Primärkörper
- 300: extrakorporalen Apparatur

## Patentansprüche

1. Vorrichtung zur extrakorporalen Temperierung von Patienten, aufweisend:
- ein Hypothermiegerät (200); und
- ein externes Temperiersystem (100) zum Temperieren einer extrakorporalen Apparatur (300), wobei das externe Temperiersystem (100) ein Leitungssystem (110) für ein Temperiermedium (157) und eine mit dem Leitungssystem (110) verbundene Befülleinrichtung (150) zum Befüllen und Entleeren des Temperiermediums in das Leitungssystem (110) aufweist; wobei die Befülleinrichtung eine Pumpe (154) zum Fördern des Temperiermediums in das Leitungssystem (110) oder aus dem Leitungssystem (110) aufweist,
wobei das Leitungssystem (110) lösbar mit dem Hypothermiegerät (200) verbunden ist und das Leitungssystem (110) eingerichtet ist zum lösbaren Verbinden mit der extrakorporalen Apparatur (300); und
wobei das Hypothermiegerät (200) dazu eingerichtet ist, ein in dem Leitungssystem (110) befindliches Temperiermedium zu temperieren; und
wobei die Befülleinrichtung (150), bevorzugt das externe Temperiersystem (100), frei von manuell betätigbaren Ventilen ist.

2. Vorrichtung nach Anspruch 1, wobei das externe Temperiersystem (100) ferner ein Rückschlagventil aufweist; und/oder wobei die Vorrichtung dazu eingerichtet ist, das Leitungssystem (110) automatisch zu Befüllen oder zu Entleeren; und/oder wobei, zum Befüllen oder Entleeren des Leitungssystems (100), die Vorrichtung dazu eingerichtet ist das Temperiermedium (157) mittels der Pumpe (154) für eine vorbestimmte Zeit zu fördern.

3. Vorrichtung nach einem der vorhergehenden Ansprüche, wobei die Befülleinrichtung (150) einen Sensor (155) aufweist, welcher dazu eingerichtet ist einen Fluss des Temperiermediums in der Befülleinrichtung (150) zu erfassen, und insbesondere wobei die Vorrichtung dazu eingerichtet ist das Befüllen oder Entleeren des Leitungssystem (110) anzuhalten falls ein Fluss in der Befülleinrichtung (150) unterhalb eines vorbestimmten Schwellenwert ist.

4. Vorrichtung nach einem der vorhergehenden Ansprüche, wobei die Befülleinrichtung (150) ein Reservoir (151) zum Bereitstellen des Temperiermediums für das externe Temperiersystem (100) aufweist, und
insbesondere wobei die Befülleinrichtung (150) dazu eingerichtet ist, das Leitungssystem (110) mit dem Temperiermedium aus dem Reservoir (151) zu befüllen und/oder das Temperiermedium aus dem Leitungssystem (110) in das Reservoir (151) zu entleeren.

5. Vorrichtung nach einem der vorhergehenden Ansprüche, wobei das Leitungssystem (110) eine Vorlaufleitung (111) und eine Rücklaufleitung (112) aufweist,
wobei die Vorlaufleitung (111) und die Rücklaufleitung (112) jeweils verbindbar mit der extrakorporalen Apparatur (300) zum Ausbilden eines externen Temperierkreislaufs sind, und
wobei das Leitungssystem (110) ferner eine Bypassleitung (116) aufweist zum Ausbilden einer Fluidverbindung zwischen der Vorlaufleitung (111) und der Rücklaufleitung (112).

6. Vorrichtung nach einem der vorhergehenden Ansprüche, wobei die Vorrichtung dazu eingerichtet ist das Leitungssystem (110) über die Bypassleitung (116) zu befüllen oder zu entleeren, ohne dass das externe Temperiersystem (100) mit der extrakorporalen Apparatur (300) verbunden sein muss oder verbunden ist; und/oder wobei die Vorrichtung dazu eingerichtet ist das Temperiermedium im Leitungssystem (110) umzuwälzen, ohne dass das externe Temperiersystem (100) mit der extrakorporalen Apparatur (300) verbunden sein muss oder verbunden ist.

7. Vorrichtung nach einem der vorhergehenden Ansprüche, wobei die Vorlaufleitung (111) und die Rücklaufleitung (112) des Leitungssystems (110) jeweils eine selbstschließende Kupplung (117, 118) aufweisen zum Anschließen an die extrakorporale Apparatur (300); und/oder wobei die Befülleinrichtung (150) eine mit dem Leitungssystem (110) verbundene Ablaufleitung (153) aufweist.

8. Vorrichtung nach einem der vorhergehenden Ansprüche, wobei das Leitungssystem (110) einen Sekundärkörper (113) aufweist, welcher dazu eingerichtet mit einem externen Primärkörper (210) des Hypothermiegeräts (200) gekoppelt zu werden; und
wobei ein Ablaufanschluss des Leitungssystems (110) an die Ablaufleitung (153) an einem höchsten Punkt des Sekundärkörpers (140), insbesondere des Leitungssystems (110), angeordnet ist.

9. Vorrichtung nach einem der vorhergehenden Ansprüche, wobei das externe Temperiersystem (100) einen in der Vorlaufleitung (111) angeordneten Temperatursensor (120) aufweist; und/oder
wobei das externe Temperiersystem (100), insbesondere das Leitungssystem (110), eine UV-Quelle (119) zum Desinfizieren des Temperiermediums aufweist, und
wobei die Vorrichtung dazu eingerichtet ist kontinuierlich Temperiermedium von der Befülleinrichtung (150) durch das Leitungssystem (110) zu zirkulieren.

10. Vorrichtung nach einem der vorhergehenden Ansprüche, wobei das externe Temperiersystem (100) ein Umschaltventil aufweist, welches einen ersten und einen zweiten Zustand aufweist,
wobei in dem ersten Zustand die Befülleinrichtung (150), insbesondere die Befüllleitung (152), mit der Vorlaufleitung (111) in Fluidverbindung eingebunden ist, und
wobei in dem zweiten Zustand die Befülleinrichtung (150), insbesondere die Befüllleitung (152), mit der Rücklaufleitung (112) in Fluidverbindung eingebunden ist.

11. Vorrichtung nach einem der vorhergehenden Ansprüche, wobei das Leitungssystem (110) eine in der Vorlaufleitung (111) angeordnete Vorlaufpumpe (114) und eine in der Rücklaufleitung (112) angeordnete Rücklaufpumpe (115) aufweist.

12. Vorrichtung nach Anspruch 11, wobei die Vorlaufpumpe (114) dazu eingerichtet ist einen Überdruck in der Vorlaufleitung (111) auszubilden und/oder wobei die Rücklaufpumpe (115) dazu eingerichtet ist einen Unterdruck in der Rücklaufleitung (112) auszubilden; und/oder wobei die Vorlaufpumpe (114) und die Rücklaufpumpe (115) jeweils eine einstellbare Förderleistung aufweisen.

13. Vorrichtung nach einem der Ansprüche 11 bis 12, wobei die Vorlaufpumpe (114) dazu eingerichtet ist einen individuell einstellbaren Über- oder Unterdruck in der Vorlaufleitung (111) auszubilden und wobei die Rücklaufpumpe (115) dazu eingerichtet ist einen individuell einstellbaren Über- oder Unterdruck in der Rücklaufleitung (112) auszubilden.

14. Verfahren zum Betreiben eines externen Temperierkreislaufs für eine extrakorporale Apparatur (300), das Verfahren umfassend die Schritte:
i) Lösbares Verbinden eines Hypothermiegeräts (200), insbesondere dem Hypothermiegerät (200) nach einem der Ansprüche 1 bis 13, mit einem externen Temperiersystem (100) zum Temperieren einer extrakorporalen Apparatur (300), insbesondere dem externen Temperiersystem (100) nach einem der Ansprüche 1 bis 13;
ii) Lösbares Verbinden des externen Temperiersystems (100) mit der extrakorporalen Apparatur (300) zum Ausbilden eines externen Temperierkreislaufs;
iii) Automatisches Befüllen des externen Temperierkreislaufs mit einem Temperiermedium mittels einer Befülleinrichtung (150), ohne manuellen Betätigens eines Ventils des externen Temperiersystems (100).

15. Verwendung eines externen Temperiersystems (100) nach einem der Ansprüche 1 bis 13 und/oder einem Hypothermiegerät (200) nach einem der Ansprüche 1 bis 13 zum Befüllen und Entleeren des Temperiermediums in das Leitungssystem (110) ohne dass Ventile des externen Temperiersystems (100) manuell betätigt werden müssen.
